# EUROPÄISCHE PATENTSCHRIFT

(11) **EP 3 493 924 B1**
(45) Veröffentlichungstag und Bekanntmachung des Hinweises auf die Patenterteilung: **22.01.2020**
(21) Anmeldenummer: 17748752.7
(22) Anmeldetag: 03.08.2017
(51) Int. Cl.: B07C 5/342, G01N 21/71, G01N 21/85, G01N 21/94, G01N 33/20

(54) **VORRICHTUNG UND VERFAHREN ZUR LEGIERUNGSANALYSE VON SCHROTTFRAGMENTEN AUS METALL**
DEVICE AND METHOD FOR ANALYSING THE ALLOY COMPOSITION OF METAL SCRAP FRAGMENTS
SYSTÈME ET PROCÉDÉ D'ANALYSE DE L'ALLIAGE DE FRAGMENTS DE DÉCHETS COMPOSÉS DE MÉTAL

(30) Priorität: 04.08.2016 DE 102016114468
(43) Veröffentlichungstag der Anmeldung: 12.06.2019
(73) Patentinhaber: Hydro Aluminium Rolled Products GmbH, 41515 Grevenbroich (DE)
(72) Erfinder: GILLNER, Ronald, 53913 Swisttal (DE); BAUERSCHLAG, Nils Robert, 52072 Aachen (DE)
(74) Vertreter: Cohausz & Florack
(86) Internationale Anmeldenummer: PCT/EP2017/069710
(87) Internationale Veröffentlichungsnummer: WO 2018/024841

(56) Entgegenhaltungen:
- DE-A1- 4 426 475
- DE-A1- 4 426 490
- DE-A1-102010 032 792
- DE-A1-102014 013 160
- US-A1- 2003 034 281

## Beschreibung

Die Erfindung betrifft eine Vorrichtung zur Legierungsanalyse an Schrottfragmenten aus Metall, mit einer Legierungsanalyseeinrichtung, die dazu eingerichtet ist, mittels einer lokalen Messung an einer Messposition auf der Oberfläche eines zu analysierenden Schrottfragments eine Zusammensetzungsinformation über die Legierungszusammensetzung des Schrottfragments zu bestimmen. Die Erfindung betrifft weiterhin ein Verfahren zur Legierungsanalyse von Schrottfragmenten aus Metall, insbesondere unter Verwendung der zuvor genannten Vorrichtung.

Im Stand der Technik erfolgt das Sortieren bzw. Recyceln von Aluminium über mehrere Verfahrensschritte. Diese umfassen in der Regel das Sammeln der unterschiedlichen Aluminiumschrotte, eine mechanische Aufbereitung der Schrotte und eine anschließende metallurgische Verwertung der Schrotte.

Für ein ressourceneffizientes Recycling sollte die mechanische Aufbereitung der Schrotte ein Aluminiumschrottprodukt erzeugen, das den qualitativen Ansprüchen des metallurgischen Verwertungsweges entspricht. Hierzu werden im Stand der Technik unterschiedliche Aufbereitungsschritte durchgeführt, die allerdings nur eine begrenzte Sortierung in Qualitäten bzw. Legierungszusammensetzungen der Schrotte erlaubt.

Die mechanische Aufbereitung erfolgt in der Regel über eine ein- oder mehrstufige Zerkleinerung der Schrotte, der sich dann diverse Sortierschritte anschließen. Die Sortierschritte können beispielsweise eine Trennung von Eisen und NE-Metallen über Magnetscheider, Windsichtung, Wirbelstromscheidung, sensorgestützte Sortierungen zum Beispiel mittels Röntgentransmission oder -fluoreszens, Induktion, laserinduzierte Plasmaspektroskopie (Laser Induced Breakdown Spectroscopy - LIBS) oder Nahinfrarotanalyse (NIR) bewirken. Die verfahrenstechnische Kombination dieser Sortierschritte erlaubt das Sortieren der Schrotte in unterschiedliche Aluminiumqualitäten, d.h. insbesondere abhängig von ihrer Legierungszusammensetzung.

Um unterschiedliche Aluminiumlegierungen legierungsspezifisch sortieren zu können, müssen Gehalte eines oder mehrerer Legierungselemente der einzelnen Schrottfragmente bestimmt werden. Hierzu werden typischerweise Systeme für laserinduzierte Plasmaspektroskopie (LIBS) oder Röntgenfluoreszenz (XRF) eingesetzt. Die mit diesen Systemen erzeugten Analyseergebnisse werden mit vorgegebenen Legierungszusammensetzungen verglichen und die jeweiligen Schrottfragmente der passenden Legierungszusammensetzung zugeordnet. Wird beispielsweise bei der Analyse eines Schrottfragments ein Mg-Gehalt von 5% ermittelt, so wird dieses Schrottfragment beispielsweise einer Klasse "Mg5-Legierung" zugeordnet.

Ein grundsätzliches Problem bei der Analyse von Schrottfragmenten und einer darauf basierenden Sortierung der Schrottfragmente sind Oberflächenverunreinigungen der einzelnen Schrottfragmente, die die Metalloberfläche der Schrottfragmente bedecken und damit eine Analyse erschweren. Bei den Oberflächenverunreinigungen kann es sich beispielsweise um Lacke (z.B. bei Schrottfragmenten von lackierten Kraftfahrzeugkomponenten), Eloxierschichten, Kunststoffschichten (z.B. bei Schrottfragmenten von Verbundmaterialien wie kunststofflaminierten Aluminiumprodukten) oder sogenannte Querverschmutzungen aus dem Sammel- und/oder Zerkleinerungsprozess handeln, die beispielsweise infolge von Restfeuchte an den Schrottfragmenten anhaften können.

Die Oberflächenverunreinigungen entsprechen in ihrer chemischen Zusammensetzung nicht der darunter liegenden Metallmatrix, insbesondere Aluminiummatrix. Diese Oberflächenverunreinigungen haben daher Auswirkungen auf Analyse- bzw. Sortiersystem, die die oberflächennahe Stoffzusammensetzung ermitteln, da die Analyseergebnisse durch die Oberflächenverunreinigungen stark verfälscht werden. Wird die Oberflächenzusammensetzung in einem verschmutzten Oberflächenbereich eines Schrottfragments bestimmt, so ergibt die Analyse anstelle der Zusammensetzung der Legierung des Schrottfragments stattdessen die chemische Zusammensetzung der Oberflächenverunreinigung oder eine Mischung aus der Zusammensetzung der Oberflächenverunreinigung und der darunter liegenden Metallmatrix. Die Analyseergebnisse sind daher nicht eindeutig bzw. stark fehlerbehaftet.

Zwar werden bei der lasergestützten Legierungsanalyse, beispielsweise LIBS, in der Regel mehrere Laserschüsse auf eine Stelle eines Schrottfragments aufgebracht, so dass eine mögliche Oberflächenverunreinigung teilweise abgetragen (ablatiert) wird. Es ist jedoch nicht gewährleistet, dass Oberflächenverunreinigungen an der Messposition vollständig verdampfen und die reine Metallmatrix freilegen, so dass die Analyseergebnisse weiterhin stark fehlerbehaftet sein können.

Aus der DE 44 26 490 A1 ist eine Vorrichtung zur Analyse von metallischen Teilen mit Laserlicht bekannt. Aus der DE 10 2014 013 160 A1 sind ein Verfahren und eine Vorrichtung zur Sortierung von wiederverwertbaren Rohstoffstücken bekannt. Aus der DE 44 26 475 A1 sind eine Anordnung und ein Verfahren zur laserinduzierten Plasmaspektroskopie bekannt. Aus der US 2003/0034281 A1 ist ein Metallschrottsortierungssystem mittels LIBS bekannt. Aus der DE 10 2010 032 792 A1 ist ein Verfahren zur Reinheitsgradbestimmung von Metallen bekannt.

Vor diesem Hintergrund liegt der vorliegenden Erfindung die Aufgabe zu Grunde, eine Vorrichtung und ein Verfahren zur Legierungsanalyse von Schrottfragmenten aus Metall zur Verfügung zu stellen, mit denen eine zuverlässigere Analyse einzelner Aluminiumfragmente ermöglicht wird.

Diese Aufgabe wird bei einer Vorrichtung gemäß Anspruch 1 zur Legierungsanalyse an Schrottfragmenten aus Metall mit einer Legierungsanalyseeinrichtung, die dazu eingerichtet ist, mittels einer ersten lokalen Messung an einer Messposition auf der Oberfläche eines zu analysierenden Schrottfragments eine Zusammensetzungsinformation über die Legierungszusammensetzung des Schrottfragments zu bestimmen, erfindungsgemäß zumindest teilweise dadurch gelöst, dass die Vorrichtung eine Verunreinigungsanalyseeinrichtung aufweist, die dazu eingerichtet ist, mittels einer zweiten lokalen Messung an der Messposition eine zugehörige Verunreinigungsinformation über die Verunreinigung der Oberfläche des Schrottfragments an der Messposition zu bestimmen, und dass die Vorrichtung eine Verarbeitungseinrichtung aufweist, die dazu eingerichtet ist, ein Analyseergebnis über die Legierungszusammensetzung des Schrottfragments abhängig von der Zusammensetzungsinformation und von der zugehörigen Verunreinigungsinformation zu bestimmen, wobei die Legierungsanalyseeinrichtung für eine laserinduzierte Plasmaspektroskopiemessung eingerichtet ist, dadurch gekennzeichnet, dass die Legierungsanalyseeinrichtung dazu eingerichtet ist, für die erste lokale Messung auf der Oberfläche des Schrottfragments ein Plasma an der Messposition zu erzeugen, und dass die Verunreinigungsanalyseeinrichtung dazu eingerichtet ist, einen Wert für eine Messgröße des von der Legierungsanalyseeinrichtung erzeugten Plasmas zu messen und abhängig von dem gemessenen Wert die zugehörige Verunreinigungsinformation zu bestimmen.

Die oben genannte Aufgabe wird erfindungsgemäß weiterhin zumindest teilweise gelöst durch ein Verfahren gemäß Anspruch 8 zur Legierungsanalyse von Schrottfragmenten aus Metall, insbesondere unter Verwendung der zuvor beschriebenen Vorrichtung, bei dem mittels einer ersten lokalen Messung an einer Messposition auf der Oberfläche eines zu analysierenden Schrottfragments eine Zusammensetzungsinformation über die Legierungszusammensetzung des Schrottfragments bestimmt wird, wobei die erste lokale Messung eine laserinduzierte Plasmaspektroskopiemessung ist, bei dem mittels einer zweiten lokalen Messung an der Messposition eine zugehörige Verunreinigungsinformation über die Verunreinigung der Oberfläche des Schrottfragments an der Messposition bestimmt wird und bei dem abhängig von der Zusammensetzungsinformation und der zugehörigen Verunreinigungsinformation ein Analyseergebnis über die Legierungszusammensetzung des Schrottfragments bestimmt wird, dadurch gekennzeichnet, dass für die erste lokale Messung auf der Oberfläche des Schrottfragments ein Plasma an der Messposition erzeugt wird und bei der zweiten lokalen Messung ein Wert für eine Messgröße des Plasmas gemessen wird und abhängig von dem gemessenen Wert die zugehörige Verunreinigungsinformation bestimmt wird, insbesondere durch Vergleich des gemessenen Werts mit einem Referenzwert.

Durch das beschriebene Verfahren bzw. mit der beschriebenen Vorrichtung kann eine mögliche Verunreinigung der Oberfläche eines analysierten Schrottfragments bei der Legierungsanalyse berücksichtigt werden. Insbesondere kann auf diese Weise durch eine separate zweite Messung festgestellt werden, ob die Zusammensetzungsinformation ausreichend gut die Legierungszusammensetzung des Schrottfragments widerspiegelt oder durch eine Oberflächenverschmutzung zu stark verfälscht ist. Dadurch kann eine zuverlässige Legierungsanalyse von Schrottfragmenten erreicht werden, mit der insbesondere eine zusammensetzungsspezifische Sortierung der Schrottfragmente ermöglicht wird.

Die Vorrichtung weist eine Legierungsanalyseeinrichtung auf, die dazu eingerichtet ist, mittels einer ersten lokalen Messung an einer Messposition auf der Oberfläche des Schrottfragments eine Zusammensetzungsinformation über die Legierungszusammensetzung des Schrottfragments zu bestimmen. Die Legierungsanalyseeinrichtung analysiert also nicht das gesamte Material des Schrottfragments, sondern nur einen Teil des Materials an einer Messposition auf der Oberfläche des Schrottfragments. Eine solche lokale Messung hat den Vorteil, dass sie schnell und relativ einfach durchgeführt werden kann. Weiterhin können für lokale Analysen effektive Messverfahren wie zum Beispiel die laserinduzierte Plasmaspektroskopie (LIBS) verwendet werden, mit denen sich die lokale Zusammensetzung eines Schrottfragments mit hoher Geschwindigkeit und ausreichender Genauigkeit bestimmen lässt.

Die Zusammensetzungsinformation über die Legierungszusammensetzung des Schrottfragments umfasst vorzugsweise einen oder mehrere Werte für den Gehalt eines oder mehrerer Legierungselemente, wie zum Beispiel Magnesium, Silizium, Eisen, Aluminium etc.

Bekannte Messverfahren zur Legierungsanalyse sind typischerweise auf bestimmte Legierungselemente beschränkt, um eine schnelle und ausreichend genaue Analyse zu ermöglichen. Daher lässt sich aus der mit einer solchen Legierungsanalyse gewonnenen Zusammensetzungsinformation nicht zuverlässig bestimmen, ob die Oberfläche des Schrottfragments an der Messposition verunreinigt ist oder nicht.

Aus diesem Grund weist die Vorrichtung weiterhin eine Verunreinigungsanalyseeinrichtung auf, die dazu eingerichtet ist, mittels einer zweiten lokalen Messung an der Messposition eine zugehörige Verunreinigungsinformation über die Verunreinigung der Oberfläche des Schrottfragments an der Messposition zu bestimmen. Die Verunreinigungsanalyseeinrichtung ist demnach in der Lage, eine Information über die Verunreinigung an der Messposition zur Verfügung zu stellen, an der von der Legierungsanalyseeinrichtung eine Messung zur Bestimmung der Zusammensetzungsinformation durchgeführt wird. Auf diese Weise erfolgt unabhängig von der Legierungsanalyse eine zusätzliche Messung, um zu bestimmen, ob eine signifikante Verunreinigung an der Messposition vorliegt und somit ob die Zusammensetzungsinformation von der Messposition zuverlässig ist oder nicht.

Unter einer Verunreinigung der Oberfläche wird vorliegend verstanden, dass das Metall des Schrottfragments unter einer Beschichtung oder einer anderweitigen Verunreinigung verborgen ist. Bei der Beschichtung kann es sich insbesondere um eine Lackschicht, eine Eloxierschicht oder eine Kunststoffschicht handeln. Bei den anderweitigen Verunreinigungen kann es sich beispielsweise um Querverschmutzungen aus dem Sammel- und/oder Zerkleinerungsprozess handeln, wie zum Beispiel Stäube oder Schmierstoffe, die an der Oberfläche des Schrottfragments anhaften. Derartige Schichten oder anderweitige Verunreinigungen führen dazu, dass die von der Legierungsanalyseeinrichtung bestimmte Zusammensetzungsinformation stark verfälscht wird. Bei der Verunreinigungsinformation kann es sich beispielsweise um eine einfache Ja/Nein-Information handeln, ob eine Verunreinigung vorliegt ("Ja") oder nicht ("Nein"). Alternativ kann die Verunreinigungsinformation auch einen Wert für den Grad der Verunreinigung enthalten, beispielsweise auf einer Skala von 0 ("nicht verunreinigt") bis 3 ("stark verunreinigt").

Die Vorrichtung weist weiterhin eine Verarbeitungseinrichtung auf, die dazu eingerichtet ist, ein Analyseergebnis über die Legierungszusammensetzung des Schrottfragments abhängig von der Zusammensetzungsinformation und von der zugehörigen Verunreinigungsinformation zu bestimmen. Die Verunreinigungsinformation wird demnach zur Bestimmung des Analyseergebnisses mitberücksichtigt. Zeigt die Verunreinigungsinformation an, dass keine oder nur eine geringe Verunreinigung an der Messposition vorliegt, so kann die Zusammensetzungsinformation als Analyseergebnis verwendet werden. Zeigt die Verunreinigungsinformation hingegen eine starke Verunreinigung an der Messstelle an, so kann als Analyseergebnis beispielsweise eine vorgegebene Information verwendet werden, dass die Analyse nicht erfolgreich war. Das Analyseergebnis kann beispielsweise ausgegeben oder zur Steuerung der Vorrichtung verwendet werden.

Das Analyseergebnis umfasst vorzugsweise einen oder mehrere Werte für den Gehalt eines oder mehrerer Legierungselemente, wie zum Beispiel Magnesium, Silizium, Eisen, Aluminium etc. auf Basis der Zusammensetzungsinformation und/oder eine Information über die Zuverlässigkeit der bestimmten Gehalte auf Basis der Verunreinigungsinformation.

Im Folgenden werden verschiedene Ausführungsformen der Vorrichtung und des Verfahrens beschrieben, wobei die einzelnen Ausführungsformen jeweils sowohl für die Vorrichtung als auch für das Verfahren anwendbar sind und sich zudem untereinander kombinieren lassen.

Bei einer ersten Ausführungsform erfolgen die erste lokale Messung und die zweite lokale Messung gleichzeitig. Auf diese Weise können die Messungen insbesondere einfacher so aufeinander abgestimmt werden, dass sie an derselben Messposition erfolgen. Insbesondere kann eine für die erste Messung bewirkte lokale Einwirkung auf das Schrottfragment, wie zum Beispiel ein an der Messposition erzeugtes Plasma, auch für die zweite Messung genutzt werden.

Die Legierungsanalyseeinrichtung ist für eine spektroskopische Messung eingerichtet, und zwar für eine laserinduzierte Plasmaspektroskopiemessung (LIBS). Bei dem Verfahren ist die erste lokale Messung eine spektroskopische Messung, und zwar eine laserinduzierte Plasmaspektroskopiemessung. Durch eine spektroskopische Messung lässt sich auf schnelle und zuverlässige Weise die Legierungszusammensetzung an der Oberfläche eines Schrottfragments bestimmen. Die laserinduzierte Plasmaspektroskopie hat sich als besonders geeignete Verfahren herausgestellt, um die Legierungszusammensetzung schnell und zuverlässig zu messen. Da das LIBS-Verfahren oberflächensensitiv ist, d.h. die Zusammensetzung nur im Oberflächenbereich des Schrottfragments bestimmt wird, wird eine LIBS-Messung stark von etwaigen Verschmutzungen der Oberfläche beeinflusst. Durch das vorliegend beschriebene Verfahren bzw. die vorliegend beschriebene Vorrichtung wird erreicht, dass sich die Ergebnisse der LIBS-Messung zuverlässiger verarbeiten lassen, da zusätzlich eine Information über die Verunreinigung an der Messposition bestimmt wird, die einen Rückschluss auf die Aussagekraft der oberflächensensitiven LIBS-Messung ermöglicht.

Die Legierungsanalyseeinrichtung ist dazu eingerichtet, für die erste lokale Messung auf der Oberfläche des Schrottfragments ein Plasma an der Messposition zu erzeugen, und die Verunreinigungsanalyseeinrichtung ist dazu eingerichtet, einen Wert für eine Messgröße des von der Legierungsanalyseeinrichtung erzeugten Plasmas zu messen und abhängig von dem gemessenen Wert die zugehörige Verunreinigungsinformation zu bestimmen. Bei dem Verfahren wird für die erste lokale Messung ein Plasma an der Messposition erzeugt, wird bei der zweiten lokalen Messung ein Wert für eine Messgröße des Plasmas gemessen und abhängig von dem gemessenen Wert die zugehörige Verunreinigungsinformation bestimmt. Unter einer Messgröße des Plasmas wird eine quantifizierbare Eigenschaft des Plasmas verstanden, die durch Messung bestimmt werden kann. Beispiele hierfür sind die Plasmafarbe, die Plasmatemperatur, die Ausdehnung des Plasmas senkrecht zur Schrottfragmentoberfläche (Plasmahöhe) oder parallel zur Schrottfragmentoberfläche (Plasmadurchmesser).

Das Plasma kann beispielsweise dadurch erzeugt werden, dass das Schrottfragment an der Messposition mit einem Laserstrahl beaufschlagt wird. Durch die Energie des Laserstrahls wird ein kleines Volumen an der Oberfläche des Schrottfragments verdampft und zumindest teilweise ionisiert, so dass an der Messposition ein lokales Plasma entsteht. Das beim Zerfall des Plasmas entstehende Licht ist für die im Plasma enthaltenen Elemente charakteristisch, so dass sich durch eine spektroskopische Untersuchung die Zusammensetzung des vom Schrottfragment abgetragenen Materials im Plasma und damit eine Zusammensetzungsinformation über die Legierungszusammensetzung des Schrottfragments bestimmen lässt. Dieses Verfahren wird bei einer LIBS-Messung angewandt.

Im Rahmen der Erfindung wurde erkannt, dass eine Verunreinigung an der Messposition zur Änderung der Eigenschaften des Plasmas führt, so dass sich durch eine zusätzliche Messung am Plasma ermitteln lässt, ob eine Verunreinigung an der Messposition vorliegt. Insbesondere beeinflusst eine Verunreinigung die Plasmafarbe, die Plasmatemperatur, den Plasmadurchmesser auf der Oberfläche, die Plasmahöhe über der Oberfläche und/oder den Strahlungsintensitätsverlauf über die Zeit. Bei der zweiten lokalen Messung kann daher insbesondere ein Wert für eine dieser Messgrößen oder für eine Kombination daraus gemessen werden.

Aus dem gemessenen Wert für die Messgröße des von der Legierungsanalyseeinrichtung erzeugten Plasmas lässt sich dann die zugehörige Verunreinigungsinformation bestimmen. Zu diesem Zweck erfolgt vorzugsweise ein Vergleich des gemessenen Werts für die Messgröße mit einem Referenzwert von einer Referenzprobe. Auf diese Weise ist keine absolute, sondern nur eine relative Auswertung des gemessenen Werts zu einem Referenzwert erforderlich. Es ist also insbesondere nicht erforderlich, die Plasmafarbe, Plasmatemperatur oder eine andere Messgröße des Plasmas absolut zu bestimmen; vielmehr ist es ausreichend, den gemessenen Wert mit einem zuvor bestimmten Referenzwert zu vergleichen, um eine Aussage über die Verunreinigung des analysierten Schrottfragments treffen zu können.

Zur Ermittlung eines entsprechenden Referenzwertes wird insbesondere eine Referenzprobe in der Vorrichtung angeordnet, mit der Legierungsanalyseeinrichtung ein Plasma auf der Oberfläche der Referenzprobe erzeugt und mit der Verunreinigungsanalyseeinrichtung ein Wert für die entsprechende Messgröße des Plasmas gemessen. Als Referenzprobe kann insbesondere ein Metallstück mit einer verunreinigungsfreien Oberfläche verwendet werden. Das Metallstück weist vorzugsweise eine den zu analysierenden Schrottfragmenten vergleichbare Zusammensetzung auf. Sollen mit der Vorrichtung beispielsweise Aluminiumschrottfragmente analysiert werden, so wird als Referenzprobe insbesondere ein Aluminiumstück mit sauberer Oberfläche verwendet.

Indem die Referenzprobe und die zu analysierenden Schrottfragmente bzw. das auf diesen erzeugte Plasma in der Vorrichtung unter im Wesentlichen gleichen Bedingungen analysiert werden, kann durch den Vergleich der Messwerte eines Schrottfragments und der Referenzprobe zuverlässig auf eine mögliche Verunreinigung des Schrottfragments an der Messposition geschlossen werden. Die Messung an der Referenzprobe dient damit insbesondere zur Kalibration der Verunreinigungsanalyseeinrichtung bzw. der zweiten lokalen Messung.

Entsprechend ist die Verunreinigungsanalyseeinrichtung bei einer bevorzugten Ausführungsform dazu eingerichtet, die zugehörige Verunreinigungsinformation mittels eines Vergleichs des gemessenen Werts für die Messgröße des Plasmas mit einem Referenzwert zu bestimmen. Bei einer entsprechenden Ausführungsform des Verfahrens wird die zugehörige Verunreinigungsinformation vorzugsweise durch Vergleich des gemessenen Werts mit einem Referenzwert bestimmt.

Bei einer weiteren Ausführungsform weist die Verunreinigungsanalyseeinrichtung eine Bilderfassungseinrichtung zur Aufnahme von Bilddaten des von der Legierungsanalyseeinrichtung an der Messposition erzeugten Plasmas auf und ist dazu eingerichtet, den Wert für die Messgröße bzw. die Verunreinigungsinformation abhängig von den Bilddaten zu bestimmen. Bei der Bilderfassungseinrichtung kann es sich beispielsweise um eine Kamera wie zum Beispiel eine CCD-Kamera handeln. Bei einer entsprechenden Ausführungsform des Verfahrens werden bei der zweiten lokalen Messung Bilddaten des an der Messposition erzeugten Plasmas aufgenommen und der Wert für die Messgröße des Plasmas bzw. die Verunreinigungsinformation wird abhängig von den Bilddaten bestimmt.

Mit einer solchen Bilderfassungseinrichtung lassen sich Werte für verschiedene Messgrößen des Plasmas wie die Plasmafarbe, die Plasmagröße (Plasmadurchmesser und/oder Plasmahöhe) oder der Strahlungsintensitätsverlauf des Plasmas über die Zeit ausreichend genau ermitteln, um damit eine Verunreinigung an der Messposition bestimmen zu können. Gegenüber aufwändigeren und empfindlicheren Messapparaturen stellt eine solche Bilderfassungseinrichtung zudem eine robuste Apparatur dar, die insbesondere für den Einsatz in einem Recyclingwerk geeignet ist.

Insbesondere wurde festgestellt, dass die Ermittlung eines Werts für eine Messgröße des Plasmas mittels einer Bilderfassungseinrichtung und ein Vergleich des gemessenen Werts mit einem an einer Referenzprobe ermittelten Wert ausreichend sind, um eine Verunreinigungsinformation zu bestimmen.

Mit einer Bilderfassungseinrichtung, insbesondere CCD-Kamera, lässt sich ein Wert für die Plasmafarbe bestimmen. Zu diesem Zweck werden die Bildpunkte der von der Bilderfassungseinrichtung hinsichtlich ihrer Farbe in einem Farbraum, beispielsweise dem RGB-Farbraum oder bevorzugt dem YCbCr-Farbraum bewertet.

Zu diesem Zweck kann beispielsweise mittels Bildverarbeitung festgestellt werden, welche Bildpunkte das Plasma abbilden. Erfolgt die Messung beispielsweise in abgedunkelter Umgebung, so kann anhand der Helligkeitswerte einfach entschieden werden, ob ein Bildpunkt Licht vom Plasma zeigt oder nicht. Ist die relative Positionierung zwischen der Messposition und der Bilderfassungseinrichtung fix, können die Bildpunkte, die das Plasma zeigen, auch vordefiniert sein.

Von den Bildpunkten, die das Plasma abbilden, wird jeweils ein Farbwert in einem vorgegebenen Farbraum bestimmt. Die Häufigkeit der Farbwerte ergibt ein Histogramm, das zur quantitativen Bewertung der Farbverteilung genutzt werden kann. Durch Vergleich des auf diese Weise erzeugten Histogramms mit einem Referenzhistogramm einer Farbverteilung eines Plasmas von einer Referenzprobe kann bestimmt werden, ob an der Messposition des analysierten Schrottfragments eine Verunreinigung vorliegt.

Aufgrund des Vergleichs mit der Referenzmessung ist es nicht erforderlich, die Plasmafarbe absolut zu bestimmen. Insbesondere muss die Messung nicht unter genormten Bedingungen (z.B. unter Normlicht mit vorgegebenem Normalbeobachter etc.) erfolgen, da es nur auf die relative Analyse ankommt, nicht aber auf absolute Farbwerte. Die Referenzmessung an der Referenzprobe soll jedoch unter im Wesentlichen gleichen Bedingungen durchgeführt werden, wie die Messungen an den zu analysierenden Schrottfragmenten, um eine möglichst aussagekräftige Analyse zu erhalten.

Mit einer Bilderfassungseinrichtung, insbesondere CCD-Kamera, lässt sich auch ein Wert für die Plasmatemperatur bestimmen. Zu diesem Zweck wird insbesondere eine Bilderfassungseinrichtung verwendet, die auf Infrarotstrahlung bzw. Nahinfrarotstrahlung sensitiv ist. Dadurch kann mit der Bilderfassungseinrichtung die Emission des Plasmas im Infrarot- bzw. Nahinfrarotbereich aufgezeichnet werden. Die das Plasma abbildenden Bildpunkte werden sodann bewertet, um einen Wert für eine gemittelte Temperatur und/oder für einen Temperaturgradienten des erzeugten Plasmas zu bestimmten. Dieser Wert kann dann mit einem entsprechenden an einer Referenzprobe ermittelten Wert verglichen werden, um die Verunreinigungsinformation zu bestimmen.

Unter der Plasmafarbe wird demnach vorliegend insbesondere die Farbe bzw. Farbverteilung verstanden, die sich aus Bilddaten des Plasmas im sichtbaren Bereich des Lichts ableiten lässt.

Unter der Plasmatemperatur wird vorliegend entsprechend insbesondere die Temperatur und/oder ein Temperaturgradient verstanden, die sich aus Bilddaten des Plasmas im infraroten bzw. nahinfraroten Bereich des Lichts ableiten lässt.

Unter dem Plasmadurchmesser auf der Oberfläche wird die größte Ausdehnung des Plasmas in einer Ebene parallel zur Oberfläche des Schrottfragments an der Messposition verstanden. Unter der Plasmahöhe über der Oberfläche wird die größte Ausdehnung des Plasmas senkrecht zur Oberfläche des Schrottfragments verstanden. Ein Wert für den Plasmadurchmesser bzw. die Plasmahöhe lässt sich ebenfalls mit einer Bilderfassungseinrichtung bestimmen. Zu diesem Zweck wird die entsprechende Ausdehnung des Plasmas (Durchmesser bzw. Höhe) aus Bilddaten des Plasmas ermittelt. So kann als Wert für den Plasmadurchmesser beispielsweise die Zahl der nebeneinanderliegenden Bildpunkte bestimmt werden, über die sich das Bild des Plasmas in den vorzugsweise von oben aufgenommen Bilddaten erstreckt. Auf analoge Weise kann ein Wert für die Plasmahöhe aus seitlich aufgenommenen Bilddaten bestimmt werden. Aufgrund des Vergleichs mit einem Referenzwert ist es nicht erforderlich, den Plasmadurchmesser bzw. die Plasmahöhe absolut zu bestimmen.

Unter dem Strahlungsintensitätsverlauf über die Zeit wird die Intensität der vom Plasma in einen definierten Raumwinkel abgestrahlten Strahlungsintensität als Funktion über die Zeit verstanden. Auch der Strahlungsintensitätsverlauf über die Zeit lässt sich auf einfache Weise aus Bilddaten vom Plasma ermitteln, beispielsweise indem für jeden Bildpunkt des Plasmas ein Helligkeitswert bestimmt und diese Helligkeitswerte zu einer momentanen Strahlungsintensität aufsummiert werden. Durch eine solche Analyse in vorgegebenen Zeitintervallen lässt sich der Strahlungsintensitätsverlauf über die Zeit einfach bestimmen.

Wie bereits ausgeführt, müssen die gemessenen Werte für die Messgrößen des Plasmas nicht absolut bestimmt werden. Es hat sich gezeigt, dass bereits ein relativer Vergleich mit einer entsprechenden Messung an einer Referenzprobe oder auch von unter gleichen Messbedingungen an verschiedenen Messpositionen gemessenen Werten für eine Messgröße des Plasmas einen Rückschluss auf die absolute und/oder relative Verunreinigung an den Messpositionen erlaubt.

Es wurde unter anderem festgestellt, dass eine Verunreinigung der Oberfläche einen Wärmetransport von der Oberfläche in das Metall des Legierungsfragments behindert. Dies führt dazu, dass bei einer Verunreinigung der Oberfläche Wärmeenergie langsamer von der Messposition abgeführt wird als ohne Verunreinigung der Oberfläche. Entsprechend kann bei einer höheren Plasmatemperatur, einem größeren Plasmadurchmesser, einer größeren Plasmahöhe und/oder einem langsamer abfallenden Strahlungsintensitätsverlauf auf eine stärkere Verunreinigung an der Messposition geschlossen werden, während eine geringere Plasmatemperatur, ein kleinerer Plasmadurchmesser, eine kleinere Plasmahöhe und/oder ein schneller abfallender Strahlungsintensitätsverlauf anzeigt, dass die Metalloberfläche des Schrottfragments an der Messposition freiliegt bzw. nur eine geringe Verunreinigung vorliegt.

Bei einer weiteren Ausführungsform sind die Legierungsanalyseeinrichtung und die Verunreinigungsanalyseeinrichtung dazu eingerichtet, eine Mehrzahl von Zusammensetzungsinformationen und zugehörigen Verunreinigungsinformationen an verschiedenen Messpositionen auf der Oberfläche eines Schrottfragments zu bestimmen, und die Verarbeitungseinrichtung ist dazu eingerichtet, abhängig von der jeweils zugeordneten Verunreinigungsinformation eine oder mehrere Zusammensetzungsinformationen aus der Mehrzahl der bestimmten Zusammensetzungsinformationen auszuwählen und aus den ausgewählten Zusammensetzungsinformationen das Analyseergebnis über die Legierungszusammensetzung des Schrottfragments zu bestimmen.

Bei einer entsprechenden Ausführungsform des Verfahrens werden eine Mehrzahl von Zusammensetzungsinformationen und zugehörigen Verunreinigungsinformationen an verschiedenen Messpositionen auf der Oberfläche eines Schrottfragments bestimmt, werden abhängig von der jeweils zugeordneten Verunreinigungsinformation eine oder mehrere Zusammensetzungsinformationen aus der Mehrzahl der bestimmten Zusammensetzungsinformationen ausgewählt und wird aus den ausgewählten Zusammensetzungsinformationen das Analyseergebnis über die Legierungszusammensetzung des Schrottfragments bestimmt.

Durch eine Mehrzahl an Messungen an verschiedenen Messpositionen auf der Oberfläche eines Schrottfragments lässt sich die Genauigkeit des Analyseergebnisses über die Legierungszusammensetzung des Schrottfragments erhöhen, insbesondere durch Mittelung von Legierungselementgehalten aus mehreren Zusammensetzungsinformationen von verschiedenen Messpositionen. Bei einer bereichsweise verunreinigten Schrottfragmentoberfläche führen Messungen an Messpositionen in verunreinigten Bereichen jedoch zu systematischen Abweichungen, die das Analyseergebnis verfälschen würden. Indem die an den verschiedenen Messpositionen ermittelten Zusammensetzungsinformationen abhängig von den jeweils zugeordneten Verunreinigungsinformationen ausgewählt werden, kann erreicht werden, dass zur Bestimmung des Analyseergebnisses nur Zusammensetzungsinformationen von Messpositionen ohne signifikante Verunreinigung verwendet werden. Werden an einem Schrottfragment beispielsweise fünf Messungen durchgeführt und zeigen die Verunreinigungsinformationen der ersten zwei Messungen eine erhöhte Verunreinigung an, so können beispielsweiseise nur die Zusammensetzungsinformationen der übrigen drei Messungen ausgewählt werden, um das Analyseergebnis zu bestimmen. Das Analyseergebnis kann dann durch eine Mittelwertbildung aus den Zusammensetzungsinformationen dieser drei Messungen bestimmt werden.

Bei einer weiteren Ausführungsform umfasst die Vorrichtung eine Fördereinrichtung zum Transport von Schrottfragmenten durch einen Analysebereich, in dem die Messung durch die Legierungsanalyseeinrichtung und die Verunreinigungsanalyseeinrichtung erfolgt. Bei einer entsprechenden Ausführungsform des Verfahrens wird ein zu analysierendes Schrottfragment durch einen Analysebereich transportiert, in dem die erste und die zweite Messung an dem Schrottfragment erfolgen. Das Verfahren und die Vorrichtung können insbesondere zur Analyse einer Menge Schrottfragmente eingesetzt werden, beispielsweise um eine Menge Schrottfragmente legierungsspezifisch zu sortieren. Mit der Fördereinrichtung können die Schrottfragmente sukzessive zur Legierungsanalyseeinrichtung und Verunreinigungsanalyseeinrichtung transportiert und dort analysiert werden. Bei der Fördereinrichtung kann es sich beispielsweise um ein Förderband handeln.

Bei einer weiteren Ausführungsform umfasst die Vorrichtung eine Vereinzelungsvorrichtung, die dazu eingerichtet ist, eine Menge Schrottfragmente zu vereinzeln, so dass die Schrottfragmente der Legierungsanalyseeinrichtung vereinzelt zugeführt werden. Bei einer entsprechenden Ausführungsform des Verfahrens wird eine bereitgestellte Menge Schrottfragmente vereinzelt und Analyseergebnisse der vereinzelten Schrottfragmente werden bestimmt. Durch die Vereinzelung der Schrottfragmente wird erreicht, dass die einzelnen Schrottfragmente zuverlässig analysiert werden können, ohne dass Schrottfragmente einander überlappen. Durch eine Vereinzelung können die Schrottfragmente zudem in einer wohldefinierten Reihenfolge analysiert werden, wodurch die Zuordnung der bestimmten Analyseergebnisse zu den einzelnen Schrottfragmenten, beispielsweise für eine nachgelagerte Sortierung der Schrottfragmente, vereinfacht wird.

Bei einer weiteren Ausführungsform umfasst die Vorrichtung eine Sortiereinrichtung, die dazu eingerichtet ist, Schrottfragmente abhängig von dem jeweils zugehörigen Analyseergebnis zu sortieren. Bei einer entsprechenden Ausführungsform des Verfahrens werden die Schrottfragmente abhängig von den für die einzelnen Schrottfragmente bestimmten Analyseergebnissen sortiert. Die für die einzelnen Schrottfragmente bestimmten Analyseergebnisse erlauben es, die Schrottfragmente legierungsspezifisch zu sortieren. Indem das Analyseergebnis nicht nur abhängig von der bestimmten Zusammensetzungsinformation, sondern auch abhängig von der zugehörigen Verunreinigungsinformation bestimmt wird, kann eine bessere Sortierung erreicht werden, so dass sich die beschriebene Legierungsanalyse insbesondere für ein Verfahren bzw. eine Vorrichtung zur Sortierung von Schrottfragmenten eignet. Beispielsweise können Schrottfragmente, für die wegen einer zu hohen Verunreinigung an der Messposition keine zuverlässige Legierungszusammensetzung bestimmt werden konnte, gezielt aussortiert werden, so dass diese Schrottfragmente nicht zu einer Verschlechterung der Sortierung führen.

Bei einer weiteren Ausführungsform weist die Vorrichtung eine Steuerungseinrichtung auf, die dazu eingerichtet ist, die Vorrichtung gemäß dem zuvor beschriebenen Verfahren bzw. einer Ausführungsform davon zu steuern. Die Steuerungseinrichtung kann beispielsweise einen Mikroprozessor und einen damit verbunden elektronischen Speicher aufweisen, wobei der Speicher Befehle enthält, deren Ausführung auf dem Mikroprozessor die Ausführung des beschriebenen Verfahrens bzw. einer Ausführungsform davon bewirkt. Auf diese Weise lässt sich die Analyse der Schrottfragmente automatisiert und zuverlässig durchführen.

Weitere Vorteile und Merkmale der Vorrichtung und des Verfahrens ergeben sich aus der nachfolgenden Beschreibung von Ausführungsbeispielen, wobei auf die beigefügte Zeichnung Bezug genommen wird.

In der Zeichnung zeigen
- Fig. 1: ein Ausführungsbeispiel der erfindungsgemäßen Vorrichtung und des erfindungsgemäßen Verfahrens in schematischer Darstellung,
- Fig. 2: die Legierungsanalyseeinrichtung und die Verunreinigungsanalyseeinrichtung der Vorrichtung aus Fig. 1,
- Fig. 3: eine Illustration der Messgröße der Plasmahöhe,
- Fig. 4: eine Illustration der Messgröße des Plasmadurchmessers und
- Fig. 5: eine schematische Darstellung einer Mehrzahl von Messungen an verschiedenen Messpositionen auf einer Schrottfragmentoberfläche.

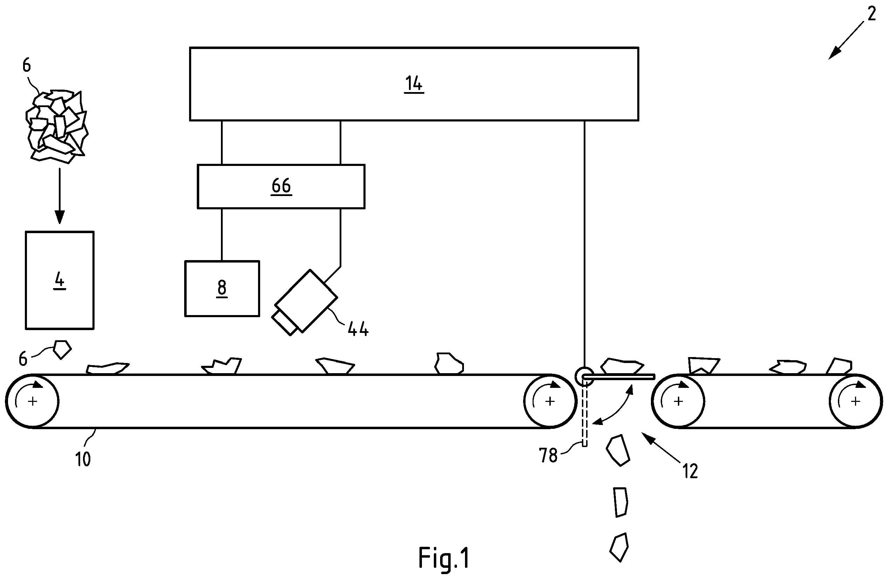

Fig. 1 zeigt ein Ausführungsbeispiel der erfindungsgemäßen Vorrichtung und des erfindungsgemäßen Verfahrens zur Legierungsanalyse von Schrottfragmenten aus Metall in schematischer Darstellung.

Die Vorrichtung 2 umfasst eine Vereinzelungseinrichtung 4 zur Vereinzelung von Schrottfragmenten 6, eine Legierungsanalyseeinrichtung 8 zur Legierungsanalyse der Schrottfragmente 6 sowie eine Fördereinrichtung 10 in Form eines Förderbands, mit dem die Schrottfragmente 6 durch die Vorrichtung 2 transportiert werden. Die Vorrichtung 2 kann wie in Fig. 1 dargestellt als Sortiervorrichtung ausgebildet sein und entsprechend eine Sortiereinrichtung 12 umfassen. Weiterhin weist die Vorrichtung noch eine Steuerungseinrichtung 14 zur Steuerung der Vorrichtung 2 auf.

Wird der Vereinzelungseinrichtung 4 eine Menge Schrottfragmente 6 zugegeben, so werden die Schrottfragmente 6 durch die Vereinzelungseinrichtung 4 vereinzelt und so auf die Fördereinrichtung 10 gegeben, dass sie einander nicht überlappen. Weiterhin weisen die Schrottfragmente 6 durch die Vereinzelung eine wohldefinierte Reihenfolge auf, wodurch die Analyse und eine mögliche anschließende Sortierung der Schrottfragmente 6 erleichtert wird.

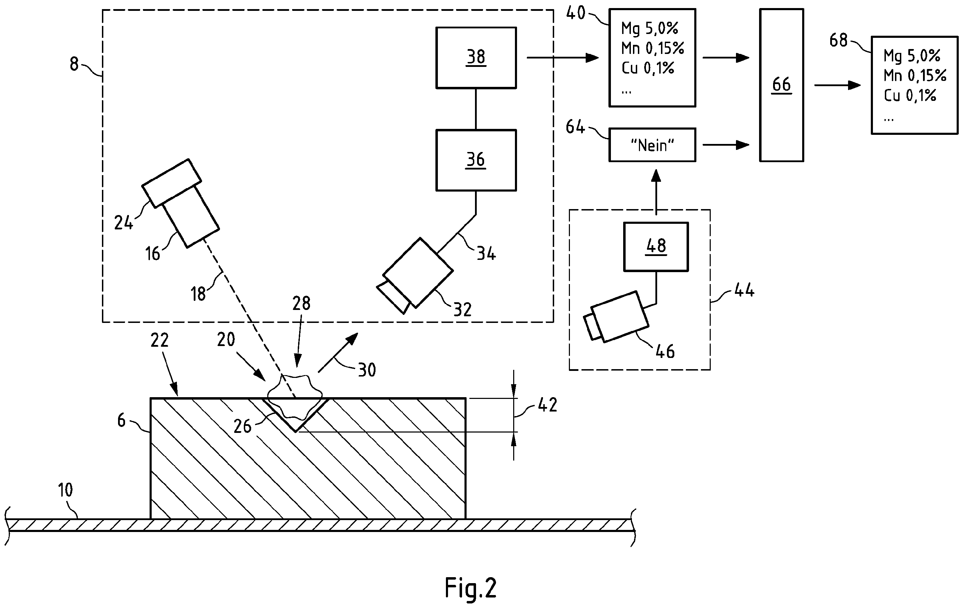

Die auf der Fördereinrichtung transportierten Schrottfragmente werden dann einzeln von der Legierungsanalyseeinrichtung 8 mittels laserinduzierter Plasmaspektroskopie untersucht, um die Legierungszusammensetzung der einzelnen Schrottfragmente 6 zu bestimmen. Der Aufbau und die Funktionsweise der Legierungsanalyseeinrichtung 8 werden im Folgenden anhand der Fig. 2 erläutert, die eine detailliertere schematische Darstellung der Legierungsanalyseeinrichtung 8 zeigt.

Die Legierungsanalyseeinrichtung 8 umfasst eine Laserquelle 16, mit der ein gepulster Laserstrahl 18 auf eine Messposition 20 auf der Oberfläche 22 eines auf der Fördereinrichtung 10 beförderten Schrottfragments 6 gerichtet werden kann. Die Legierungsanalyseeinrichtung 8 kann eine Positionierungseinrichtung 24 umfassen, mit der sich der Laserstrahl 18 ausrichten lässt. Auf diese Weise kann die Messposition 20 auf der Oberfläche 22 gezielt ausgewählt werden.

Durch den auf der Oberfläche 22 auftreffenden Laserstrahl 18 wird an der Messposition 22 ein kleines Volumen 26 im Bereich der Oberfläche des Schrottfragments 6 verdampft und zu einem Plasma 28 ionisiert. Beim Zerfall des Plasmas 28 wird Licht 30 emittiert, das charakteristisch für die im Volumen 26 enthaltenen Legierungselemente ist. Durch eine spektroskopische Analyse des Lichts 30 lassen sich daher die Legierungselemente im Plasma 28 bestimmen und damit auf die Legierungszusammensetzung des Volumens 26 rückschließen.

Entsprechend weist die Legierungsanalyseeinrichtung 8 weiterhin eine Optik 32 auf, um das Licht 30 einzufangen und über einen Lichtleiter 34 an ein Spektrometer 36 weiterzuleiten, mit dem die Spektralverteilung des Lichts 30 analysiert werden kann. Eine an das Spektrometer angeschlossene Auswerteelektronik 38 berechnet dann aus der gemessenen Spektralverteilung die Legierungszusammensetzung des Volumens 26 und gibt eine entsprechende Zusammensetzungsinformation 40 aus, die insbesondere die Gehalte bestimmter Legierungselemente wie zum Beispiel Mg, Mn, Cu etc. im Volumen 26 enthalten kann.

Da der Laserstrahl 18 auf eine bestimmte Messposition 20 gerichtet ist und zudem nur eine bestimmte Eindringtiefe 42 hat, die abhängig von der eingestellten Laserleistung typischerweise im Bereich von 1 bis 100 µm liegt, führt die Legierungsanalyseeinrichtung 8 also eine lokale Messung an einer Messposition 20 auf der Oberfläche 22 des Schrottfragments 6 durch.

Befinden sich an der Messposition 20 Verunreinigungen wie zum Beispiel eine Lackschicht, eine Kunststoffschicht oder Schmierstoffe, so wird die Messung hierdurch stark verfälscht, so dass die Zusammensetzungsinformation 40 keine zuverlässigen Informationen über die tatsächliche Legierungszusammensetzung des Schrottfragments 6 liefert.

Aus diesem Grund weist die in Fig. 1 gezeigte Vorrichtung 2 zusätzlich eine Verunreinigungsanalyseeinrichtung 44 auf, die ebenfalls in Fig. 2 dargestellt ist. Die Verunreinigungsanalyseeinrichtung 44 umfasst eine CCD-Kamera 46 und eine Auswerteeinrichtung 48. Die Kamera 46 ist so ausgerichtet, dass sie Bilddaten von dem mit der Legierungsanalyseeinrichtung 8 auf der Oberfläche 22 erzeugte Plasma 28 aufnehmen kann. Mit der Auswerteeinrichtung 48 lassen sich aus diesen Bilddaten Werte für verschiedene Messgrößen des Plasmas 28 bestimmen, insbesondere für die Plasmafarbe oder die Plasmagröße, wie zum Beispiel den Plasmadurchmesser oder die Plasmahöhe.

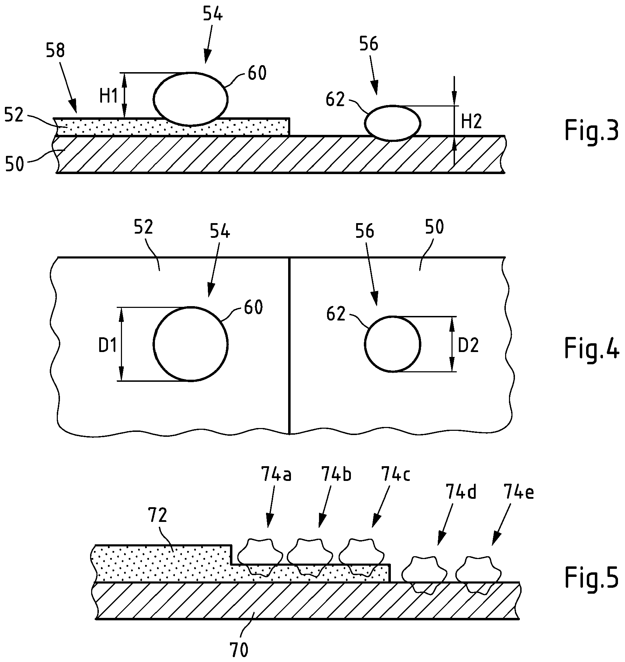

Anhand der Figuren 3 und 4 wird im Folgenden exemplarisch die Bestimmung eines Werts für die Plasmahöhe bzw. den Plasmadurchmesser aus von einer Kamera wie der Kamera 46 aufgenommenen Bilddaten erläutert. Fig. 3 zeigt ein Schrottfragment 50 von der Seite und Fig. 4 zeigt das Schrottfragment 50 von oben. Das Schrottfragment 50 weist eine partielle Lackierung 52 auf, die vorliegend eine Verunreinigung darstellt, da sie die Legierungsanalyse des Schrottfragments 50 durch eine oberflächliche Messung behindert.

Mit der Legierungsanalyseeinrichtung 8 werden Messungen an zwei verschiedenen Messpositionen 54, 56 auf der Oberfläche 58 des Schrottfragments 50 durchgeführt. Die erste Messposition 54 befindet sich im Bereich der partiellen Lackierung 52, d.h. die Oberfläche 58 ist an der ersten Messposition verunreinigt. An der zweiten Messposition 56 liegt die Metalloberfläche des Schrottfragmente 50 hingegen frei; die Oberfläche 58 weist an dieser Messposition 56 also keine Verunreinigung auf.

Für die beiden Messungen mit der Legierungsanalyseeinrichtung 8 wird die Oberfläche 58 des Schrottfragments 50 nacheinander mit dem Laserstrahl 18 beaufschlagt, so dass an den Messpositionen jeweils oberflächlich Material verdampft und sich ein Plasma 60 bzw. 62 bildet. Die beiden Plasmen 60 und 62 sind in den Fig. 3 und 4 nur zu Illustrationszwecken gleichzeitig dargestellt; tatsächlich werden sie nacheinander erzeugt.

Wie aus den Fig. 3 und 4 ersichtlich, weist das Plasma 60 an der ersten Messposition 54 eine größere Plasmahöhe H1 und einen größeren Plasmadurchmesser D1 auf als das Plasma 62 an der zweiten Messposition 56. Dies kann darauf zurückgeführt werden, dass die Lackschicht 52 den Wärmeabtransport vom Laserstrahl 18 über das Metall des Schrottfragments 50 behindert, so dass sich im Plasma 60 eine größere Energie ansammelt und das Plasma dadurch größer ist. Durch einen relativen Vergleich der Plasmahöhe H1 mit der Plasmahöhe H2 des Plasmas 62 bzw. des Plasmadurchmessers D1 mit dem Plasmadurchmesser D2 des Plasmas 62 lässt sich feststellen, dass die Oberfläche 58 an der ersten Messposition 54 eine höhere Verunreinigung aufweist als an der zweiten Messposition 56.

Die Plasmahöhe und der Plasmadurchmesser können aus den in Fig. 3 und 4 illustrierten Bilddaten beispielsweise dadurch bestimmt werden, dass die Anzahl der Pixel bestimmt wird, über die sich die Leuchterscheinung des Plasmas erstreckt.

In entsprechender Weise kann mit der Auswerteeinrichtung 48 ein Wert für die Plasmahöhe und/oder den Plasmadurchmesser des Plasmas 28 bestimmt werden. Auf Basis der bestimmten Werte für die Messgrößen des Plasmas 28 bestimmt die Analyseeinrichtung 48 sodann eine Verunreinigungsinformation 64 über die Verunreinigung an der Messposition. Die Verunreinigungsinformation 64 kann beispielsweise wie in Fig. 2 illustriert eine einfache Ja/Nein-Information sein, aus der sich ergibt, ob ein bestimmter Verunreinigungsgrad überschritten wurde ("Ja") oder nicht ("Nein"). Alternativ kann die Verunreinigungsinformation 64 auch eine Zahl zurückgeben, die den Grad der Verunreinigung an der Messposition repräsentiert.

Die mit der Legierungsanalyseeinrichtung 8 bestimmte Zusammensetzungsinformation 40 und die von der Verunreinigungsanalyseeinrichtung 44 bestimmte Verunreinigungsinformation 64 werden an eine Verarbeitungseinrichtung 66 der Vorrichtung 2 übermittelt, die aus der Zusammensetzungsinformation 40 und der Verunreinigungsinformation 64 ein Analyseergebnis 68 bestimmt. Zeigt beispielsweise die Verunreinigungsinformation 64 an, dass die Zusammensetzungsinformation 40 an einer verunreinigungsarmen oder verunreinigungsfreien Messposition bestimmt wurde, so kann die Verarbeitungseinrichtung 66 die Zusammensetzungsinformation als Analyseergebnis 68 weitergeben. Zeigt die Verunreinigungsinformation 64 hingegen an, dass die Zusammensetzungsinformation 40 an einer verunreinigten Messposition bestimmt wurde, so kann die Verarbeitungseinrichtung 66 als Analyseergebnis 68 ausgeben, dass die Analyse nicht erfolgreich war.

Die Legierungsanalyseeinrichtung 8 kann insbesondere auch dazu eingerichtet sein, mehrere Messungen an verschiedenen Messpositionen auf der Oberfläche eines Schrottfragments durchzuführen. Fig. 5 zeigt exemplarisch ein Schrottfragment 70 mit teilflächiger Lackierung 72, an der mit der Legierungsanalyseeinrichtung 8 nacheinander fünf Messungen an fünf verschiedenen Messpositionen 74a-e durchgeführt und entsprechend fünf Zusammensetzungsinformationen bestimmt werden. Mit der Verunreinigungsanalyseeinrichtung 44 werden zudem fünf zugehörige Verunreinigungsinformationen bestimmt.

Die ersten drei Messpositionen 74a-c sind im Bereich der Lackierung 72 angeordnet. Zwar wurde ein Teil der Lackierung 72 durch den Laserstrahl 18 abgetragen. Dennoch verfälscht die verbliebene Lackierung 72 die für diese Messpositionen 74a-c bestimmten Zusammensetzungsinformationen. An der vierten und fünften Messposition 74d-c liegt die Metalloberfläche des Schrottfragments 70 hingegen frei, so dass die dort bestimmten Zusammensetzungsinformationen die Zusammensetzung des Schrottfragments 70 repräsentieren.

Die Verarbeitungseinrichtung 66 ist nun vorzugsweise so eingerichtet sein, dass sie aus den fünf bestimmten Zusammensetzungsinformationen anhand der zugehörigen Verunreinigungsinformationen die besten Zusammensetzungsinformationen auswählt. Da die Verarbeitungseinrichtung 66 den Verunreinigungsinformationen entnehmen kann, dass an der vierten und fünften Messposition 74d-c keine Verunreinigung vorliegt, wählt die Verarbeitungseinrichtung 66 die zugehörige vierte und fünfte Zusammensetzungsinformation aus und bestimmt aus diesen das Analyseergebnis, insbesondere durch Mittelung der bestimmten Gehalte von Legierungselementen.

Auf diese Weise wird eine zuverlässige Legierungsanalyse von Schrottfragmenten ermöglicht.

Das Analyseergebnis 68 kann beispielsweise genutzt werden, um die Legierungszusammensetzung einer Menge Schrottfragmente 6 zu ermitteln. Zu diesem Zweck können die Schrottfragmete 6 nacheinander mit der Legierungsanalyseeinrichtung 8 analysiert werden und aus den jeweils an den einzelnen Schrottfragmenten 6 bestimmten Analyseergebnissen 68 kann die mittlere Legierungszusammensetzung der Menge Schrottfragmente 6 bestimmt werden.

Weiterhin kann das Analyseergebnis 68 auch zur Sortierung der Schrottfragmente 6 verwendet werden, indem die optionale Sortiereinrichtung 12 von der Steuerungseinrichtung 14 abhängig vom Analyseergebnis 68 gesteuert wird.

Die Sortiereinrichtung 12 ist in Fig. 1 exemplarisch als ansteuerbare, zwischen einer geschlossenen Stellung (durchgezogene Linie) und einer geöffneten Stellung (gestrichelte Linie) bewegbare Klappe 78 dargestellt. Die Klappe 78 kann durch die Sortiereinrichtung 12 beispielsweise so angesteuert werden, dass sich die Klappe 78 in die geöffnete Stellung bewegt, wenn der Gehalt einer Legierungskomponente, z.B. Mg, aus dem Analyseergebnis 68 oberhalb eines vorgegebenen Grenzwertes liegt, und dass sich die Klappe 78 in die geschlossene Stellung bewegt, wenn der Gehalt der Legierungskomponente unterhalb des vorgegebenen Grenzwertes liegt. Damit lassen sich die Schrottfragmente abhängig vom zugehörigen Analyseergebnis legierungsspezifisch sortieren.

Die ansteuerbare Klappe 78 ist nur ein einfaches Beispiel für die Sortiereinrichtung 12. Stattdessen können auch andere Einrichtungen zum Sortieren der Schrottfragmente 6 vorgesehen sein. Beispielsweise können die einzelnen Schrottfragmente auch pneumatisch sortiert werden, indem die Schrottfragmente durch einen kurzen, starken Luftstoß aussortiert werden, wenn sie ein bestimmtes Kriterium bezüglich ihrer Zusammensetzung erfüllen. Beispielsweise lassen sich auf diese Weise Schrottfragmente 6 aussortieren, bei denen das Analyseergebnis 68 anzeigt, dass keine verwertbare Legierungszusammensetzung bestimmt werden konnte.

## Patentansprüche

1. Vorrichtung (2) zur Legierungsanalyse von Schrottfragmenten (6, 50, 70) aus Metall,
- mit einer Legierungsanalyseeinrichtung (8), die dazu eingerichtet ist, mittels einer ersten lokalen Messung an einer Messposition (20, 54, 56, 74a-e) auf der Oberfläche (22, 58) eines zu analysierenden Schrottfragments (6, 50, 70) eine Zusammensetzungsinformation (40) über die Legierungszusammensetzung des Schrottfragments (6, 50, 70) zu bestimmen,
- mit einer Verunreinigungsanalyseeinrichtung (44), die dazu eingerichtet ist, mittels einer zweiten lokalen Messung an der Messposition (20, 54, 56, 74a-e) eine zugehörige Verunreinigungsinformation (64) über die Verunreinigung der Oberfläche (22, 58) des Schrottfragments (6, 50, 70) an der Messposition (20, 54, 56, 74a-e) zu bestimmen, und
- mit einer Verarbeitungseinrichtung (66), die dazu eingerichtet ist, ein Analyseergebnis (68) über die Legierungszusammensetzung des Schrottfragments (6, 50, 70) abhängig von der Zusammensetzungsinformation (40) und von der zugehörigen Verunreinigungsinformation (64) zu bestimmen,
- wobei die Legierungsanalyseeinrichtung (8) für eine laserinduzierte Plasmaspektroskopiemessung eingerichtet ist,
**dadurch gekennzeichnet,**
- **dass** die Legierungsanalyseeinrichtung (8) dazu eingerichtet ist, für die erste lokale Messung auf der Oberfläche (22, 58) des Schrottfragments (6, 50, 70) ein Plasma (28, 60, 62) an der Messposition (20, 54, 56, 74a-e) zu erzeugen, und
- **dass** die Verunreinigungsanalyseeinrichtung (44) dazu eingerichtet ist, einen Wert für eine Messgröße des von der Legierungsanalyseeinrichtung (8) erzeugten Plasmas (28, 60, 62) zu messen und abhängig von dem gemessenen Wert die zugehörige Verunreinigungsinformation (64) zu bestimmen.

2. Vorrichtung nach Anspruch 1,
**dadurch gekennzeichnet, dass** die Verunreinigungsanalyseeinrichtung (44) dazu eingerichtet ist, die zugehörige Verunreinigungsinformation (64) mittels eines Vergleichs des gemessenen Werts für die Messgröße des Plasmas (28, 60, 62) mit einem Referenzwert zu bestimmen.

3. Vorrichtung nach Anspruch 1 oder 2,
**dadurch gekennzeichnet, dass** die Messgröße des Plasmas (28, 60, 62) die Plasmafarbe, die Plasmatemperatur, der Plasmadurchmesser auf der Oberfläche, die Plasmahöhe über der Oberfläche, der Strahlungsintensitätsverlauf über die Zeit oder eine Kombination daraus ist.

4. Vorrichtung nach einem der Ansprüche 1 bis 3,
**dadurch gekennzeichnet dass** die Verunreinigungsanalyseeinrichtung (44) eine Bilderfassungseinrichtung (46) zur Aufnahme von Bilddaten des von der Legierungsanalyseeinrichtung (8) an der Messposition (20, 54, 56, 74a-e) erzeugten Plasmas (28, 60, 62) aufweist und dazu eingerichtet ist, den Wert für die Messgröße abhängig von den Bilddaten zu bestimmen.

5. Vorrichtung nach einem der Ansprüche 1 bis 4,
**dadurch gekennzeichnet,**
- **dass** die Legierungsanalyseeinrichtung (8) und die Verunreinigungsanalyseeinrichtung (44) dazu eingerichtet sind, eine Mehrzahl von Zusammensetzungsinformationen (40) und zugehörigen Verunreinigungsinformationen (64) an verschiedenen Messpositionen (20, 54, 56, 74a-e) auf der Oberfläche (22, 58) eines Schrottfragments (6, 50, 70) zu bestimmen, und
- **dass** die Verarbeitungseinrichtung (66) dazu eingerichtet ist, abhängig von der jeweils zugeordneten Verunreinigungsinformation (64) eine oder mehrere Zusammensetzungsinformationen (40) aus der Mehrzahl der bestimmten Zusammensetzungsinformationen (40) auszuwählen und aus den ausgewählten Zusammensetzungsinformationen (40) das Analyseergebnis (68) über die Legierungszusammensetzung des Schrottfragments (6, 50, 70) zu bestimmen.

6. Vorrichtung nach einem der Ansprüche 1 bis 5, aufweisend eine Sortiereinrichtung (12), die dazu eingerichtet ist, Schrottfragmente (6, 50, 70) abhängig von dem jeweils zugehörigen Analyseergebnis (68) zu sortieren.

7. Vorrichtung nach einem der Ansprüche 1 bis 6, aufweisend eine Steuerungseinrichtung (14), die dazu eingerichtet ist, die Vorrichtung (2) gemäß einem Verfahren nach einem der Ansprüche 8 bis 13 zu steuern.

8. Verfahren zur Legierungsanalyse von Schrottfragmenten (6, 50, 70) aus Metall,
- bei dem mittels einer ersten lokalen Messung an einer Messposition (20, 54, 56, 74a-e) auf der Oberfläche (22, 58) eines zu analysierenden Schrottfragments (6, 50, 70) eine Zusammensetzungsinformation (40) über die Legierungszusammensetzung des Schrottfragments (6, 50, 70) bestimmt wird, wobei die erste lokale Messung eine laserinduzierte Plasmaspektroskopiemessung ist,
- bei dem mittels einer zweiten lokalen Messung an der Messposition (20, 54, 56, 74a-e) eine zugehörige Verunreinigungsinformation (64) über die Verunreinigung der Oberfläche (22, 58) des Schrottfragments (6, 50, 70) an der Messposition (20, 54, 56, 74a-e) bestimmt wird und
- bei dem abhängig von der Zusammensetzungsinformation (40) und der zugehörigen Verunreinigungsinformation (64) ein Analyseergebnis (68) über die Legierungszusammensetzung des Schrottfragments (6, 50, 70) bestimmt wird,
**dadurch gekennzeichnet,**
- **dass** für die erste lokale Messung auf der Oberfläche (22, 58) des Schrottfragments (6, 50, 70) ein Plasma (28, 60, 62) an der Messposition (20, 54, 56, 74a-e) erzeugt wird und bei der zweiten lokalen Messung ein Wert für eine Messgröße des Plasmas (28, 60, 62) gemessen wird und abhängig von dem gemessenen Wert die zugehörige Verunreinigungsinformation (64) bestimmt wird, insbesondere durch Vergleich des gemessenen Werts mit einem Referenzwert.

9. Verfahren nach Anspruch 8,
**dadurch gekennzeichnet, dass** die erste lokale Messung und die zweite lokale Messung gleichzeitig erfolgen.

10. Verfahren nach Anspruch 8 oder 9,
**dadurch gekennzeichnet,**
- **dass** eine Mehrzahl von Zusammensetzungsinformationen (40) und zugehörigen Verunreinigungsinformationen (64) an verschiedenen Messpositionen (20, 54, 56, 74a-e) auf der Oberfläche (22, 58) eines Schrottfragments (6, 50, 70) bestimmt werden,
- **dass** abhängig von der jeweils zugeordneten Verunreinigungsinformation (64) eine oder mehrere Zusammensetzungsinformationen (40) aus der Mehrzahl der bestimmten Zusammensetzungsinformationen (40) ausgewählt werden und
- **dass** aus den ausgewählten Zusammensetzungsinformationen (40) das Analyseergebnis (68) über die Legierungszusammensetzung des Schrottfragments (6, 50, 70) bestimmt wird.

11. Verfahren nach einem der Ansprüche 8 bis 10,
**dadurch gekennzeichnet, dass** eine bereitgestellte Menge Schrottfragmente (6, 50, 70) vereinzelt wird und Analyseergebnisse (68) der vereinzelten Schrottfragmente (6, 50, 70) bestimmt werden.

12. Verfahren nach einem der Ansprüche 8 bis 11,
**dadurch gekennzeichnet, dass** die Schrottfragmente (6, 50, 70) abhängig von dem jeweils bestimmten Analyseergebnis (68) sortiert werden.

13. Verfahren nach einem der Ansprüche 8 bis 12,
**dadurch gekennzeichnet, dass** das Verfahren unter Verwendung einer Vorrichtung nach einem der Ansprüche 1 bis 7 durchgeführt wird.

## Claims

1. Apparatus (2) for analysing the alloy composition of metal scrap fragments (6, 50, 70),
- having an alloy analysing system (8) configured to determine, by means of a first local measurement at a measurement position (20, 54, 56, 74a-e) on the surface (22, 58) of a scrap fragment (6, 50, 70) to be analysed, a piece of composition information (40) about the alloy composition of the scrap fragment (6, 50, 70),
- having a contamination analysing system (44) configured to determine, by means of a second local measurement at the measurement position (20, 54, 56, 74a-e), an associated piece of contamination information (64) about the contamination of the surface (22, 58) of the scrap fragment (6, 50, 70) at the measurement position, and
- having a processing system (66) configured to determine an analysis result (68) about the alloy composition of the scrap fragment (6, 50, 70) as a function of the piece of composition information (40) and the associated piece of contamination information (64),
- wherein the alloy analysing system (8) is configured for a laser-induced plasma spectroscopy measurement,
**characterised**
- **in that** the alloy analysing system (8) is configured to generate, for the first local measurement, a plasma (28, 60, 62) on the surface (22, 58) of the scrap fragment (6, 50, 70) at the measurement position (20, 54, 56, 74a-e), and
- **in that** the contamination analysing system (44) is configured to measure a value for a measured variable of the plasma (28, 60, 62) generated by the alloy analysing system (8) and to determine the associated piece of contamination information (64) as a function of the measured value.

2. Apparatus according to claim 1, **characterised in that** the contamination analysing system (44) is configured to determine the associated piece of contamination information (64) by means of a comparison of the measured value for the measured variable of the plasma (28, 60, 62) with a reference value.

3. Apparatus according to claim 1 or claim 2, **characterised in that** the measured variable of the plasma (28, 60, 62) is the plasma colour, the plasma temperature, the plasma diameter on the surface, the plasma height above the surface, the radiation intensity profile over time or a combination thereof.

4. Apparatus according to any one of claims 1 to 3, **characterised in that** the contamination analysing system (44) has an image capturing system (46) for recording image data of the plasma (28, 60, 62) generated by the alloy analysing system (8) at the measurement position (20, 54, 56, 74a-e) and is configured to determine the value for the measurement variable as a function of the image data.

5. Apparatus according to any one of claims 1 to 4, **characterised**
- **in that** the alloy analysing system (8) and the contamination analysing system (44) are configured to determine a plurality of pieces of composition information (40) and associated pieces of contamination information (64) at various measurement positions (20, 54, 56,74a-e) on the surface (22, 58) of a scrap fragment (6, 50, 70), and
- **in that** the processing system (66) is configured to select, as a function of the respectively associated piece of contamination information (64), one or more pieces of composition information (40) from the determined plurality of pieces of composition information (40) and to determine the analysis result (68) about the alloy composition of the scrap fragment (6, 50, 70) from the selected pieces of composition information (40).

6. Apparatus according to any one of claims 1 to 5, having a sorting apparatus (12) configured to sort scrap fragments (6, 50, 70) as a function of the respectively associated analysis result (68).

7. Apparatus according to any one of claims 1 to 6, having a controller (14) configured to control the apparatus (2) according to a method according to any one of claims 8 to 13.

8. Method for analysing the alloy composition of metal scrap fragments (6, 50, 70),
- in which, by means of a first local measurement at a measurement position (20, 54, 56, 74a-e) on the surface (22, 58) of a scrap fragment (6, 50, 70) to be analysed, a piece of composition information (40) about the alloy composition of the scrap fragment (6, 50, 70) is determined, wherein the first local measurement is a laser-induced plasma spectroscopy measurement,
- in which, by means of a second local measurement at the measurement position (20, 54, 56, 74a-e), an associated piece of contamination information (64) about the contamination of the surface (22, 58) of the scrap fragment (6, 50, 70) at the measurement position (20, 54, 56, 74a-e) is determined, and
- in which an analysis result (68) about the alloy composition of the scrap fragment (6, 50, 70) is determined as a function of the piece of composition information (40) and the associated piece of contamination information (64),
**characterised**
- **in that,** for the first local measurement, a plasma (28, 60, 62) is generated on the surface (22, 58) of the scrap fragment (6, 50, 70) at the measurement position (20, 54, 56, 74a-e) and a value for a measurement variable of the plasma (28, 60, 62) is measured at the second local measurement and the associated piece of contamination information (64) is determined as a function of the measured value, in particular by comparison of the measured value with a reference value.

9. Method according to claim 8, **characterised in that** the first local measurement and the second local measurement take place simultaneously.

10. Method according to claim 8 or claim 9, **characterised**
- **in that** a plurality of pieces of composition information (40) and associated pieces of contamination information (64) are determined at various measurement positions (20, 54, 56, 74a-e) on the surface (22, 58) of a scrap fragment (6, 50, 70),
- **in that** one or more pieces of composition information (40) is or are selected from the plurality of determined pieces of composition information (40) as a function of the respectively associated pieces of contamination information (64), and
- **in that** the analysis result (68) about the alloy composition of the scrap fragment (6, 50, 70) is determined from the selected pieces of composition information (40).

11. Method according to any one of claims 8 to 10, **characterised in that** a provided quantity of scrap fragments (6, 50, 70) is separated and analysis results (68) of the separated scrap fragments (6, 50, 70) are determined.

12. Method according to any one of claims 8 to 11, **characterised in that** the scrap fragments (6, 50, 70) are sorted as a function of the respectively determined analytical result (68).

13. Method according to any one of claims 8 to 12, **characterised in that** the method is performed using an apparatus according to any one of claims 1 to 7.

## Revendications

1. Dispositif (2) d'analyse d'alliage de fragments de déchets (6, 50, 70) en métal,
- avec un dispositif d'analyse d'alliage (8) conçu pour déterminer une information de composition (40) concernant la composition d'alliage du fragment de déchets (6, 50, 70) par une première mesure locale à une position de mesure (20, 54, 56, 74a-e) à la surface (22, 58) d'un fragment de déchets (6, 50, 70) à analyser,
- avec un dispositif d'analyse de contaminations (44) conçu pour déterminer une information associée liée à des contaminations (64), concernant les contaminations de la surface (22, 58) du fragment de déchets (6, 50, 70) à la position de mesure (20, 54, 56, 74a-e), par une deuxième mesure locale à la position de mesure (20, 54, 56, 74a-e), et
- avec un dispositif de traitement (66) conçu pour déterminer un résultat d'analyse (68) concernant la composition de l'alliage du fragment de déchets (6, 50, 70) en fonction de l'information de composition (40) et de l'information associée liée à des contaminations (64),
- le dispositif d'analyse d'alliage (8) étant configuré pour une spectroscopie d'émission atomique de plasma induit par laser,
**caractérisé en ce que**
- le dispositif d'analyse d'alliage (8) est conçu pour générer, pour la première mesure locale à la surface (22, 58) du fragment de déchets (6, 50, 70), un plasma (28, 60, 62) à la position de mesure (20, 54, 56, 74a-e) et
- le dispositif d'analyse de contaminations (44) est conçu pour mesurer une valeur pour une unité de mesure du plasma (28, 60, 62) généré par le dispositif d'analyse d'alliage (8) et pour déterminer, en fonction de la valeur mesurée, l'information associée liée à des contaminations (64).

2. Dispositif selon la revendication 1, **caractérisé en ce que** le dispositif d'analyse de contaminations (44) est conçu pour déterminer l'information associée liée à des contaminations (64) par comparaison de la valeur mesurée pour l'unité de mesure du plasma (28, 60, 62) avec une valeur de référence.

3. Dispositif selon la revendication 1 ou 2, **caractérisé en ce que** l'unité de mesure du plasma (28, 60, 62) est la couleur du plasma, la température du plasma, le diamètre du plasma sur la surface, la hauteur du plasma au-dessus de la surface, l'évolution de l'intensité de rayonnement dans le temps ou une combinaison de ceux-ci.

4. Dispositif selon l'une des revendications 1 à 3, **caractérisé en ce que** le dispositif d'analyse de contaminations (44) présente un dispositif d'acquisition d'images (46) pour l'enregistrement de données d'images du plasma (28, 60, 62) généré par le dispositif d'analyse d'alliage (8) à la position de mesure (20, 54, 56, 74a-e) et est conçu pour déterminer la valeur pour l'unité de mesure en fonction des données d'images.

5. Dispositif selon l'une des revendications 1 à 4, **caractérisé en ce que**
- le dispositif d'analyse d'alliage (8) et le dispositif d'analyse de contaminations (44) sont conçus pour déterminer une pluralité d'informations de composition (40) et d'informations associées liées à des contaminations (64) à différentes positions de mesure (20, 54, 56, 74a-e) sur la surface (22, 58) d'un fragment de déchets (6, 50, 70), et
- le dispositif de traitement (66) est conçu pour sélectionner, en fonction de l'information respective associée liée à des contaminations (64), une ou plusieurs informations de composition (40) à partir de la pluralité d'informations de composition (40) déterminées et pour déterminer, à partir des informations de composition (40) sélectionnées, le résultat d'analyse (68) concernant la composition d'alliage du fragment de déchets (6, 50, 70).

6. Dispositif selon l'une des revendications 1 à 5, présentant un dispositif de triage (12) qui est conçu de sorte à trier des fragments de déchets (6, 50, 70) en fonction du résultat d'analyse (68) respectivement associé.

7. Dispositif selon l'une des revendications 1 à 6, présentant un dispositif de commande (14) qui est conçu de sorte à commander le dispositif (2) conformément à un procédé selon l'une des revendications 8 à 13.

8. Procédé d'analyse d'alliage de fragments de déchets (6, 50, 70) en métal,
- dans lequel une information de composition (40) concernant la composition d'alliage du fragment de déchets (6, 50, 70) est déterminée par une première mesure locale à une position de mesure (20, 54, 56, 74a-e) à la surface (22, 58) d'un fragment de déchets (6, 50, 70) à analyser,
la première mesure locale étant une spectroscopie d'émission atomique de plasma induit par laser,
- dans lequel une information associée liée à des contaminations (64) concernant les contaminations de la surface (22, 58) du fragment de déchets (6, 50, 70) à la position de mesure (20, 54, 56, 74a-e) est déterminée par une deuxième mesure locale à la position de mesure (20, 54, 56, 74a-e), et
- dans lequel un résultat d'analyse (68) concernant la composition de l'alliage du fragment de déchets (6, 50, 70) est déterminé en fonction de l'information de composition (40) et de l'information associée liée à des contaminations (64),
**caractérisé en ce que,**
- pour la première mesure locale à la surface (22, 58) du fragment de déchets (6, 50, 70), un plasma (28, 60,62) est généré à la position de mesure (20, 54, 56, 74a-e) et, lors de la deuxième mesure locale, une valeur pour une unité de mesure du plasma (28, 60, 62) est mesurée et, en fonction de la valeur mesurée, l'information associée liée à des contaminations (64) est déterminée, en particulier par comparaison de la valeur mesurée avec une valeur de référence.

9. Procédé selon la revendication 8, **caractérisé en ce que** la première mesure locale et la deuxième mesure locale ont lieu en même temps.

10. Procédé selon la revendication 8 ou 9,**caractérisé en ce qu'**
- une pluralité d'informations de composition (40) et d'informations associées liées à des contaminations (64) sont déterminées à différentes positions de mesure (20, 54, 56, 74a-e) à la surface (22, 58) d'un fragment de déchets (6, 50, 70),
- en fonction respectivement de l'information associée liée à des contaminations (64), une ou plusieurs informations de composition (40) sont sélectionnées parmi la pluralité d'informations de composition (40) déterminées, et
- le résultat d'analyse (68) concernant la composition d'alliage du fragment de déchets (6, 50, 70) est déterminé à partir des informations de composition (40) sélectionnées.

11. Procédé selon l'une des revendications 8 à 10, **caractérisé en ce qu'**une quantité mise à disposition de fragments de déchets (6, 50, 70) est séparée et que des résultats d'analyse (68) des fragments de déchets (6, 50, 70) séparés sont déterminés.

12. Procédé selon l'une des revendications 8 à 11, **caractérisé en ce que** les fragments de déchets (6, 50, 70) sont triés en fonction du résultat d'analyse (68) respectivement déterminé.

13. Procédé selon l'une des revendications 8 à 12, **caractérisé en ce que** le procédé est exécuté en utilisant un dispositif selon l'une des revendications 1 à 7.
